# EUROPEAN PATENT APPLICATION

(11) **EP 2 641 905 A1**
(43) Date of publication of application: **25.09.2013**
(21) Application number: 12382107.6
(22) Date of filing: 23.03.2012
(51) Int. Cl.: C07D 333/38

(54) **Solid forms of strontium ranelate and processes for their preparation**

(71) Applicant: Urquima S.A., 08184 Palau Solità I Plegamans (ES)
(72) Inventor: Prohens Lopez, Rafel, 08450 LLINARS DEL VALLÈS (ES); Puigjaner Vallet, Maria Cristina, 08029 BARCELONA (ES); Barbas Canero, Rafael, 08924 SANTA COLOMA DE GRAMENET (ES); Del Rio Pericacho, José Luis, 08225 TERRASSA (ES); MartiÍ Via, Josep, 08023 BARCELONA (ES)
(74) Representative: ZBM Patents

(57) **Abstract**

It relates to strontium ranelate in a hydrated form, having a water content of from 14 to 19% ± 0.3% by weight, in particular in the form of a mixture of crystalline Form X and crystalline Hydrate-1; to a process for its preparation; to a pharmaceutical composition comprising it; and to its use in the treatment of osteoporosis and arthrosis. It also relates to strontium ranelate in a hydrated form, having a water content of from 12.7 to 13.3% ± 0.3% by weight; in particular in crystalline Form X, and to a process for its preparation.

## Description

The present invention relates to strontium ranelate in the form of a mixture of two crystalline forms, to a process for its preparation, to a pharmaceutical composition comprising it, and to its use in medicine. It also relates to strontium ranelate in a new crystalline form, and to a process for its preparation.

### BACKGROUND ART

Strontium ranelate is the distrontium salt of 5-[bis(carboxymethyl)amino]-3-carboxymethyl-4-cyano-2-thiophenecarboxylic acid and is represented by the structural formula (I):

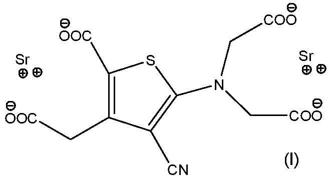

Strontium ranelate has very valuable pharmacological and therapeutic properties, especially remarkable anti-osteoporotic properties, making it useful in the treatment of bone diseases, in particular osteoporosis and arthrosis.

The preparation and the therapeutic use of strontium ranelate and its tetrahydrate, heptahydrate and octahydrate forms have been described in the European patent specification EP 415850.

EP 1642897 discloses the crystalline Form α of strontium ranelate characterized by PXRD and having a water content of 22-24%. According to the experimental data filed by the opponent on 14.07.2009 during the opposition of EP 1642897, Form α corresponds to a mixture of two hydrate crystalline forms, denominated Hydrate-1 and Hydrate-2, both of them characterized by PXRD in said document.

WO 2010/034806 discloses an anhydrous amorphous form and three hydrate crystalline forms (Form I, Form II and Form III) of strontium ranelate having a water content of less than about 5.5% and characterized by PXRD, and a process for their preparation.

IN 2010CH01267 discloses the crystalline Form A of strontium ranelate having a water content of 1.5 to 2.5% and characterized by PXRD, and a process for its preparation.

IN 2010CH03829 discloses the monohydrate crystalline Form F of strontium ranelate characterized by PXRD and having a water content of 3.0 to 4.5%, and a process for its preparation.

WO 2011/107454 discloses an anhydrous crystalline form of strontium ranelate characterized by PXRD, and a process for its preparation.

CN 101108845 discloses a process for the preparation of the heptahydrate form of strontium ranelate having a water content of 19.0-20.4% and characterized by PXRD. This polymorph seems to correspond to the previous mentioned polymorph Hydrate-1.

Although several crystalline forms of strontium ranelate are known in the art, these forms show drawbacks that render the preparation of pharmaceutical compositions comprising them difficult and disadvantageous.

Thus, strontium ranelate having a higher water content becomes a completely unmanageable, wet, sticky mass. On the other hand, strontium ranelate having a lower water content is highly hygroscopic and can easily absorb some water from the environment. In both cases, such compounds are particularly unsuitable for handling, storing and using in the preparation of pharmaceutical dosage form.

The different solid forms of a pharmaceutically active ingredient can have different characteristics, and offer certain advantages, for example with regard to stability, bioavailability, ease of formulation, ease of administration, among others. Since some solid forms are more adequate for one type of formulation, and other forms for other different formulations, the development of new solid forms allows for improving the characteristics of the pharmaceutical formulations comprising them. In addition, depending on the therapeutic indications, one or another pharmaceutical formulation may be preferred.

Especially desirable improvements/advantages of the new strontium ranelate polymorphs form would include:
- improvement of physicochemical properties in order to facilitate its manufacture or its formulation
- to enhance the absorption and/or the bioavailability
- being easily obtainable with more constant physicochemical properties
- allowing more flexibility while formulating, or facilitating its formulation,
- having better dispersibility properties, thus allowing better dispersion rates, especially if dispersed in an aqueous physiological surrounding, or
- reducing hygroscopicity;
- improving stability;
- allowing new routes of administration;

Most desirably the new polymorphs should combine more than one, or even most of these advantages.

Thus, there is a need to develop new solid forms of strontium ranelate which are suitable for use in the pharmaceutical industry and, in particular, allow easy production of strontium ranelate preparations in solid form meeting strict pharmaceutical standards, such as powders, for oral administration.

### SUMMARY OF THE INVENTION

The inventors have surprisingly found new forms of strontium ranelate, as crystalline Form X, and as a mixture of crystalline Form X and crystalline Hydrate-1, which show good stability and favourable physico-mechanical properties, while maintaining the efficacy of the known form.

In particular, strontium ranelate which is a mixture of crystalline Form X and crystalline Hydrate-1 shows a lower sedimentation rate when administered in the form of a suspension in comparison to other known strontium ranelate forms, such as strontium ranelate crystalline Hydrate-1. As a consequence, the need of redispersion of sedimented particles of the mixture in the suspension is decreased, which facilitates the administration of uniform doses.

In addition, the strontium ranelate which is a mixture of crystalline Form X and crystalline Hydrate-1 shows high polymorphic stability. As it will be shown in the examples, whereas strontium ranelate which is a mixture of crystalline Form X and crystalline Hydrate-1 having a water content of 15.1 % ± 0.3% by weight does not change its polymorphic form after four months at room temperature (20-25°C) in a closed vial, strontium ranelate as crystalline Hydrate-1 is not stable under the same conditions and it transforms into a mixture of Hydrate-1 and a further polymorphic form, Hydrate-2.

Another advantage of the solid forms of the present invention lies in the fact that they can be prepared with a simple and reproducible method, with high yields and easy industrialization.

Therefore, an aspect of the invention relates to strontium ranelate in a hydrated form, having a water content of from 14 to 19% ± 0.3% by weight; in particular, in the form of a mixture of crystalline Form X and crystalline Hydrate-1, wherein crystalline Form X shows a X-ray diffractogram that comprises characteristic peaks at 8.8, 9.0, 10.8, 13.1, 14.3, 14.7, 15.0, 17.6, 19.8, 25.0 ± 0.2 degrees 2 theta measured in a X-ray diffractometer with Cu Kα radiation (1.5418 A), and crystalline Hydrate-1 shows a X-ray diffractogram that comprises characteristic peaks at 7.9, 8.4, 9.0, 11.0, 13.2, 15.8, 18.0, 19.2, 22.8, 24.0 ± 0.2 degrees 2 theta measured in a X-ray diffractometer with Cu Kα radiation (1.5418 A).

Another aspect of the invention relates to a process for the preparation of the crystalline strontium ranelate in the form of the mixture as defined above, comprising:
a) providing strontium ranelate, having a X-ray diffractogram that comprises characteristic peaks at 7.6, 8.4, 8.6, 9.0, 13.1, 18.0, 19.1, 22.8, 23.1, 24.1, 25.6, 26.9, 28.2, 30.1 degrees 2 theta measured in a X-ray diffractometer with Cu Kα radiation (1.5406 A); and
b) drying the product of the previous step under vacuum at 20-25 °C until a water content of from 14 to 19% ± 0.3% by weight.

Another aspect of the invention relates to a pharmaceutical composition comprising a therapeutically effective amount of strontium ranelate in the form of the mixture as previously defined, together with one or more pharmaceutically acceptable excipients or carriers.

Another aspect of the invention relates to strontium ranelate in the form of the mixture as defined above, for use as a medicament, in particular in the treatment of bone diseases. This aspect may also be formulated as the use of strontium ranelate in the form of the mixture as defined above for the manufacture of a medicament for the treatment of bone diseases. The present invention also relates to a method for the treatment of bone diseases, comprising administering a therapeutically effective amount of strontium ranelate in the form of the mixture as defined above, together with pharmaceutically acceptable excipients or carriers, in a subject in need thereof, including a human.

Another aspect of the invention relates to strontium ranelate in a hydrated form, having a water content of from 12.7 to 13.3% ± 0.3% by weight; in particular in crystalline Form X, which shows a X-ray diffractogram that comprises characteristic peaks at 8.8, 9.0, 10.8, 13.1, 14.3, 14.5, 14.7, 15.0, 15.6, 17.6, 19.8, 21.5, 21.8, 22.6, 23.6, 24.3, 25.0, 26.5, 26.8, 27.1, 28.2, 29.3, 30.0, 31.6 ± 0.2 degrees 2 theta measured in a X-ray diffractometer with Cu Kα radiation (1.5418 A).

Another aspect of the invention relates to a process for the preparation of the strontium ranelate in crystalline Form X, comprising:
a) providing strontium ranelate, having a X-ray diffractogram that comprises characteristic peaks at 7.6, 8.4, 8.6, 9.0, 13.1, 18.0, 19.1, 22.8, 23.1, 24.1, 25.6, 26.9, 28.2, 30.1 degrees 2 theta measured in a X-ray diffractometer with Cu Kα radiation (1.5406 A); and
b) drying the product of the previous step under vacuum at 20-25 °C until a water content of from 12.7 to 13.3% ± 0.3% by weight.

### BRIEF DESCRIPTION OF THE DRAWINGS

FIG. 1 shows the powder X-ray diffraction pattern (intensity (counts) vs. 2-theta angle (°)) of strontium ranelate in crystalline Form X.
FIG. 2 shows the powder X-ray diffraction pattern (intensity (counts) vs. 2-theta angle (°)) of strontium ranelate as crystalline Hydrate-1, known from the prior art.
FIG. 3 shows the powder X-ray diffraction pattern (intensity (counts) vs. 2theta angle (°)) of strontium ranelate in form of a mixture of crystalline Form X and crystalline Hydrate-1, having a water content of 16.2% ± 0.3% by weight.
FIG. 4 shows the powder X-ray diffraction pattern (intensity (counts) vs. 2theta angle (°)) of strontium ranelate in form of a mixture of crystalline Form X and crystalline Hydrate-1, having a water content of 15.1 % ± 0.3% by weight.

### DETAILED DESCRIPTION OF THE INVENTION

As mentioned above, the present invention relates to strontium ranelate in a hydrated form, having a water content of from 14 to 19% ± 0.3% by weight, in particular in the form of a mixture of crystalline Form X and crystalline Hydrate-1.

The term "hydrated form" as used herein means a product having water molecules forming part of its crystalline structure. Additionally, any product of the present invention in a hydrated form may also contain water molecules not forming part of its crystalline structure.

For the purposes of the invention, the water content is determined by Karl Fisher titrimetry (KFT) as is expressed in % by weight (water weight / sample weight). In a preferred embodiment, the water content of strontium ranelate in form of a mixture as described above is of from 15 to 17% ± 0.3% by weight. More preferably, the water content is of from 15 to 16% ± 0.3% by weight, even more preferably, the water content is 15.1 % ± 0.3% by weight or 16.2% ± 0.3% by weight.

In one embodiment, the invention relates to strontium ranelate in form of a mixture of crystalline Form X and crystalline Hydrate-1, having a water content of from 14 to 19% ± 0.3% by weight, wherein crystalline Form X shows a X-ray diffractogram that comprises characteristic peaks at 8.8, 9.0, 10.8, 13.1, 14.3, 14.7, 15.0, 17.6, 19.8, 25.0 ± 0.2 degrees 2 theta measured in a X-ray diffractometer with Cu Kα radiation (1.5418 A), and crystalline Hydrate-1 shows a X-ray diffractogram that comprises characteristic peaks at 7.9, 8.4, 9.0, 11.0, 13.2, 15.8, 18.0, 19.2, 22.8, 24.0 ± 0.2 degrees 2 theta measured in a X-ray diffractometer with Cu Kα radiation (1.5418 A).

In the mixture as defined above either crystalline Form X or crystalline Hydrate-1 can be the predominant form. The term "predominant form" means that is present in the mixture as the main form, that is in a ratio higher than 50% by weight in the mixture in respect of the other form.

The term "percentage (%) by weight" refers to the percentage of each crystalline Form in relation to the total weight.

Thus, in one embodiment of the invention, in the above mixture, crystalline Form X is present in an amount of from 60 to 80% by weight and crystalline Hydrate-1 is present in an amount of from 20 to 40% by weight, being the sum of both crystalline forms 100% by weight. In a particular embodiment, crystalline Form X is present in an amount of 70% by weight and crystalline Hydrate-1 is present in an amount of 30% by weight. In a more particular embodiment, crystalline Form X is present in an amount of 70% by weight and crystalline Hydrate-1 is present in an amount of 30% by weight, and the water content is 15.1 % ± 0.3% by weight.

In another embodiment, in the above mixture, crystalline Form X is present in an amount of from 20 to 40% by weight and crystalline Hydrate-1 is present in an amount of from 60 to 80% by weight, being the sum of both crystalline forms 100% by weight. In a particular embodiment, crystalline Form X is present in an amount of 30% by weight and crystalline Hydrate-1 is present in an amount of 70% by weight. In a more particular embodiment, crystalline Form X is present in an amount of 30% by weight and crystalline Hydrate-1 is present in an amount of 70% by weight, and the water content is 16.2% ± 0.3% by weight.

For the purposes of the invention, any ranges given include both the lower and the upper end-points of the range.

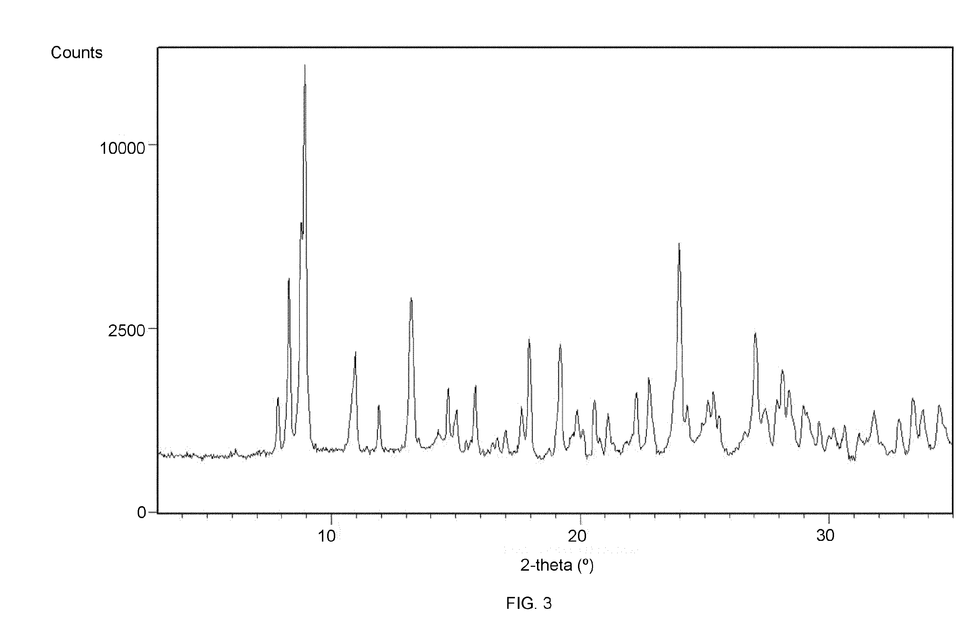

In another embodiment, crystalline Hydrate-1 is the predominant form in the mixture as defined above, and the mixture shows a X-ray diffractogram that comprises characteristic peaks at 7.8, 8.3, 8.8, 8.9, 11.0, 13.2, 17.6, 18.0, 19.2, 24.0 ± 0.2 degrees 2 theta measured in a X-ray diffractometer with Cu Kα radiation (1.5418 A). In a particular embodiment, strontium ranelate in the form of the mixture as defined above, wherein crystalline Hydrate-1 is the predominant form, is characterized by having a X-ray diffractogram as shown in FIG. 3. This diffractogram differs from the ones of other solid forms of strontium ranelate known in the state of the art. In a more particular embodiment, strontium ranelate in the form of the mixture as defined above, wherein crystalline Hydrate-1 is the predominant form, shows a X-ray diffractogram that comprises characteristic peaks at the values shown in Table 1 ± 0.2 degrees 2 theta measured in a X-ray diffractometer with Cu Kα radiation (1.5418 A).

**Table 1**

| 2-theta degrees | Intensity (%) |
|---|---|
| 7.8 | 4.9 |
| 8.3 | 26.8 |
| 8.8 | 38.6 |
| 8.9 | 100.0 |
| 11.0 | 11.1 |
| 11.9 | 4.1 |
| 13.2 | 21.5 |
| 14.3 | 1.5 |
| 14.7 | 6.2 |
| 15.0 | 3.6 |
| 15.8 | 6.4 |
| 17.6 | 4.0 |
| 18.0 | 13.6 |
| 19.2 | 12.8 |
| 19.9 | 3.8 |
| 20.6 | 4.8 |
| 21.1 | 3.1 |
| 22.3 | 5.8 |
| 22.8 | 7.4 |
| 24.0 | 35.3 |
| 24.3 | 4.3 |
| 25.2 | 4.4 |
| 25.4 | 5.8 |
| 27.0 | 14.8 |
| 27.5 | 4.0 |
| 27.9 | 4.7 |
| 28.1 | 8.7 |
| 28.4 | 6.0 |
| 29.0 | 4.1 |
| 29.6 | 2.7 |
| 30.6 | 2.4 |

In another particular embodiment, strontium ranelate in the form of the mixture as defined above, wherein crystalline Hydrate-1 is the predominant form, has a water content of 16.2 ± 0.3% by weight.

In another particular embodiment, strontium ranelate in the form of the mixture as defined above, wherein crystalline Hydrate-1 is the predominant form, shows a weight loss 18.0 ± 2% by weight in the Thermal Gravimetric Analysis (TGA).

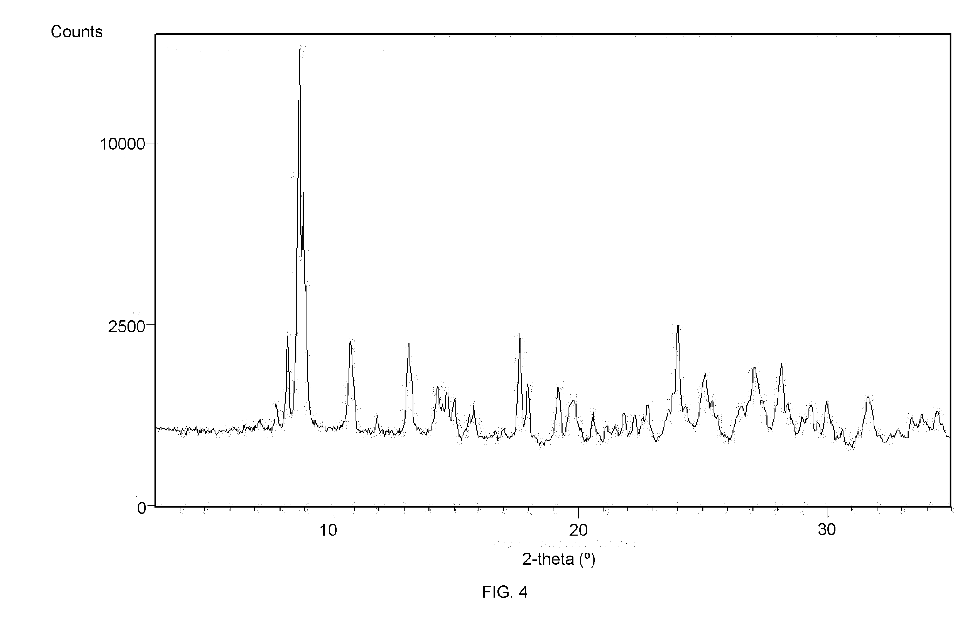

In another embodiment, crystalline Form X is the predominant form in the mixture as defined above, and the mixture shows a X-ray diffractogram that comprises characteristic peaks at 7.9, 8.3, 8.8, 9.0, 9.1, 10.8, 13.2, 17.6, 18.0, 24.0 ± 0.2 ± 0.2 degrees 2 theta measured in a X-ray diffractometer with Cu Kα radiation (1.5418 A). In a particular embodiment, strontium ranelate in the form of the mixture as defined above, wherein crystalline Form X is the predominant form, is characterized by having a X-ray diffractogram as shown in FIG. 4. This diffractogram differs from the ones of other solid forms of strontium ranelate known in the state of the art. In another particular embodiment, strontium ranelate in the form of the mixture as defined above, wherein crystalline Form X is the predominant form, shows a X-ray diffractogram that comprises characteristic peaks at the values shown in Table 2 ± 0.2 degrees 2 theta measured in a X-ray diffractometer with Cu Kα radiation (1.5418 A).

**Table 2**

| 2-theta degrees | Intensity (%) |
|---|---|
| 7.9 | 2.3 |
| 8.3 | 11.7 |
| 8.8 | 100.0 |
| 9.0 | 45.2 |
| 9.1 | 18.2 |
| 10.8 | 10.5 |
| 11.9 | 1.2 |
| 13.2 | 10.2 |
| 14.3 | 4.6 |
| 14.7 | 3.9 |
| 15.0 | 3.3 |
| 15.8 | 2.7 |
| 17.6 | 12.5 |
| 18.0 | 5.2 |
| 19.2 | 5.0 |
| 19.9 | 3.2 |
| 20.6 | 1.9 |
| 21.1 | 1.2 |
| 21.8 | 2.3 |
| 22.3 | 2.1 |
| 22.8 | 3.0 |
| 24.0 | 14.3 |
| 25.1 | 6.5 |
| 26.6 | 2.9 |
| 27.1 | 7.4 |
| 28.2 | 8.1 |
| 29.0 | 2.2 |
| 29.4 | 3.1 |
| 30.0 | 3.6 |

In another particular embodiment, strontium ranelate in the form of the mixture as defined above, wherein crystalline Form X is the predominant form, has a water content of 15.1 ± 0.3% by weight.

In another particular embodiment, strontium ranelate in the form of the mixture as defined above, wherein crystalline Form X is the predominant form, shows a weight loss 15.2 ± 2 % by weight in the Thermal Gravimetric Analysis (TGA).

The invention also provides a process for the preparation of the crystalline strontium ranelate in the form of the mixture of crystalline Form X and crystalline Hydrate-1 as defined above. This process comprises:
a) providing strontium ranelate, obtainable by the process as defined in example 1 second method (d) of EP 415850; and
b) drying the product of the previous step under vacuum at 20-25 °C until a water content of from 14 to 19% ± 0.3% by weight.

In another embodiment, strontium ranelate obtainable by the process as defined in example 1 second method (d) of EP 415850 can also be defined as strontium ranelate obtainable by the process comprising:
i) providing a solution of 2-[N,N-di(carboxymethyl)amino]-3-cyano-4-carboxymethylthiophene-5-carboxylic acid tetrasodium salt in water;
ii) reacting the solution obtained in step a) with a solution of strontium chloride in water; and
iii) isolating the product from step ii) from the reaction medium.

In one embodiment of the process above, the strontium chloride is provided in a two fold molar excess in respect of the tetrasodium salt.

Strontium ranelate obtainable by the process as defined in example 1 second method (d) of EP 415850 has a X-ray diffractogram that comprises characteristic peaks at 7.6, 8.4, 8.6, 9.0, 13.1, 18.0, 19.1, 22.8, 23.1, 24.1, 25.6, 26.9, 28.2, 30.1 degrees 2 theta measured in a X-ray diffractometer with Cu Kα radiation (1.5406 A).

In one embodiment, strontium ranelate obtainable by the process as defined in example 1 second method (d) of EP 415850 has a X-ray diffractogram as identified in the experimental data filed by the opponent on 14.07.2009 during the opposition of EP 1642897, and in particular shows a X-ray diffractogram that comprises characteristic peaks at the values shown in Table 3 degrees 2 theta measured in a X-ray diffractometer with Cu Kα radiation (1.5406 A).

**Table 3**

| 2theta (°) | I/I₀ [%] |
|---|---|
| 7.6 | 29.4 |
| 8.0 | 13.2 |
| 8.4 | 21.1 |
| 8.6 | 62.9 |
| 9.0 | 19.8 |
| 10.9 | 5.1 |
| 11.3 | 11.5 |
| 12.0 | 14.4 |
| 13.1 | 15.7 |
| 13.6 | 6.6 |
| 14.2 | 1.8 |
| 14.7 | 4.8 |
| 14.8 | 9.6 |
| 15.8 | 9.7 |
| 16.1 | 14 |
| 16.7 | 9 |
| 17.3 | 11 |
| 17.6 | 5.4 |
| 18.0 | 16.2 |
| 19.1 | 19.2 |
| 20.0 | 12.7 |
| 20.4 | 3.8 |
| 20.8 | 13 |
| 21.3 | 11.4 |
| 21.7 | 2.4 |
| 22.4 | 10.9 |
| 22.8 | 21.5 |
| 23.1 | 18.6 |
| 23.6 | 7.6 |
| 24.1 | 100 |
| 24.8 | 3.9 |
| 25.1 | 11.4 |
| 25.6 | 25.7 |
| 26.0 | 7.2 |
| 26.5 | 7.1 |
| 26.9 | 35.7 |
| 27.7 | 12.1 |
| 28.2 | 49.2 |
| 29.1 | 19 |
| 29.7 | 11.7 |
| 30.1 | 16.6 |
| 32.0 | 13.2 |
| 32.6 | 1.4 |
| 32.8 | 6.1 |
| 33.3 | 12.1 |

Generally, the drying step of the above process is carried out for the period of time which is necessary to complete the conversion of the starting solid form into crystalline strontium ranelate in the form of the mixture as defined above, that is, until the water content of the resulting product is of from 14 to 19% ± 0.3% by weight. The time needed depends on the vacuum used. The skilled person would know how to adjust the parameters of this process in the light of the description and examples of the present invention.

In a particular embodiment, the process above for the preparation of crystalline strontium ranelate in the form of the mixture as previously defined further comprises the steps of:
i) washing the product of step a) with water;
ii) optionally washing the product of step i) with an organic solvent; and.
iii) isolating the product obtained in the previous step from the medium.

For the purposes of the invention the term "washing with a solvent" means mixing the product with a solvent.

In a more particular embodiment, the amount of water used in step i) is of from 2 to 10 volumes, more particularly of from 2 to 5 volumes.

In another particular embodiment, the process comprises carrying out step ii), more particularly the organic solvent of step ii) is acetone.

Generally, step iii) can be carried out by several methods know in the art, such as evaporation, filtration or centrifugation. In one embodiment, the isolation is carried out by filtration.

The present invention also relates to a pharmaceutical composition comprising a therapeutically effective amount of strontium ranelate in the form of the mixture as defined above, together with one or more pharmaceutically acceptable excipients or carriers.

In one embodiment, the pharmaceutical composition as defined above is for oral, parenteral (intravenous or subcutaneous) or nasal administration. In another embodiment, the pharmaceutical composition as defined above is for oral administration.

The expression "pharmaceutically acceptable excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

The expression "therapeutically effective amount" as used herein, refers to the amount of a compound that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations.

Another aspect of the invention relates to strontium ranelate in the form of the mixture as defined above, for use as a medicament, in particular, for use in the treatment of a bone disease. In a preferred embodiment, the bone disease is osteoporosis or arthrosis.

Strontium ranelate in form of a mixture as defined above is composed of crystalline Form X and crystalline Hydrate-1. Crystalline Form X forms also part of the invention.

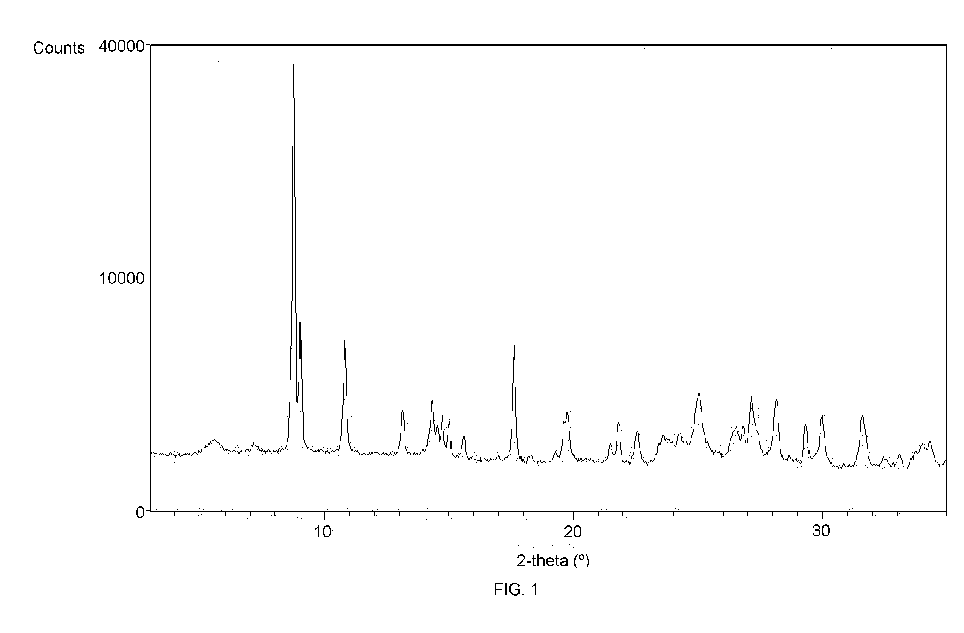

As mentioned above, the present invention relates to strontium ranelate in a hydrated form, having a water content of from 12.7 to 13.3% ± 0.3% by weight; in particular in crystalline Form X. In one embodiment, strontium ranelate in crystalline Form X shows a X-ray diffractogram that comprises characteristic peaks at 8.8, 9.0, 10.8, 13.1, 14.3, 14.7, 15.0, 17.6, 19.8, 25.0 ± 0.2 degrees 2 theta measured in a X-ray diffractometer with Cu Kα radiation (1.5418 A). In one embodiment of the invention, strontium ranelate in crystalline Form X is characterized by having a X-ray diffractogram as shown in FIG. 1. In another embodiment, strontium ranelate in crystalline Form X, has a X-ray diffractogram that comprises characteristic peaks at the values shown in Table 4 ± 0.2 degrees 2 theta measured in a X-ray diffractometer with Cu Kα radiation (1.5418 Å).

**Table 4**

| 2-theta degrees | Intensity (%) |
|---|---|
| 8.8 | 100.0 |
| 9.0 | 17.1 |
| 10.8 | 13.0 |
| 13.1 | 3.5 |
| 14.3 | 4.7 |
| 14.5 | 2.3 |
| 14.7 | 3.2 |
| 15.0 | 2.7 |
| 15.6 | 1.5 |
| 17.6 | 12.7 |
| 19.8 | 3.6 |
| 21.5 | 1.1 |
| 21.8 | 2.8 |
| 22.6 | 2.0 |
| 23.6 | 1.7 |
| 24.3 | 1.9 |
| 25.0 | 5.8 |
| 26.5 | 2.4 |
| 26.8 | 2.6 |
| 27.1 | 5.6 |
| 28.2 | 5.2 |
| 29.3 | 2.9 |
| 30.0 | 3.6 |
| 31.6 | 3.7 |

In another embodiment, strontium ranelate in crystalline Form X has a water content of from 13.3% ± 0.3% by weight.

Strontium ranelate crystalline Form X can be prepared by the process comprising:
a) providing strontium ranelate, obtainable by the process as defined in example 1 second method (d) of EP 415850; and
b) drying the product of the previous step under vacuum at 20-25 °C until a water content of from 12.7 to 13.3% ± 0.3% by weight.

In a first particular embodiment, the above process for the preparation of strontium ranelate crystalline Form X further comprises the steps of:
i) providing a suspension of the product of step a) in an organic solvent; and
ii) isolating the product of step i) from the medium.

In a more particular embodiment, step i) is performed with organic solvent, which is selected from the group consisting of (C₁-C₆)alcohols, (C₂-C₄)nitriles, (C₃-C₈)ketones, (C₃-C₆)amides, (C₂-C₄)sulfoxides, and cyclic (C₃-C₆)ethers. In another embodiment, the organic solvent is selected from the group consisting of ethanol, 2-propanol, acetonitrile, acetone, dimethylformamide, dimethylsulfoxide, tetrahydrofuran, and dioxane, at a temperature comprised of from 0 °C to the reflux temperature of the solvent, preferably at room temperature (20-25 °C).

In a second particular embodiment, the above process for the preparation of strontium ranelate crystalline Form X further comprises the steps of:
i') washing the product of step a) with water;
ii') optionally washing the product of step i') with an organic solvent; and.
iii') isolating the product obtained in the previous step from the medium.

In a more particular embodiment, the amount of water used in step i') is of from 2 to 10 volumes, more particularly of from 2 to 5 volumes.

In another particular embodiment, the process comprises carrying out step ii'), more particularly the organic solvent of step ii') is acetone.

Generally, step ii) or step iii') of the processes above can be carried out by several methods know in the art, such as evaporation, filtration or centrifugation. In one embodiment, the isolation is carried out by filtration.

Hydrate-1 is a known form of strontium ranelate, characterized by having a X-ray diffractogram that comprises characteristic peaks at 7.9, 8.4, 9.0, 11.0, 13.2, 15.8, 18.0, 19.2, 22.8, 24.0 ± 0.2 degrees 2 theta measured in a X-ray diffractometer with Cu Kα radiation (1.5418 A). In one embodiment, strontium ranelate crystalline Hydrate-1 is characterized by having a X-ray diffractogram that comprises characteristic peaks at the values shown in Table 5 ± 0.2 degrees 2 theta measured in a X-ray diffractometer with Cu Kα radiation (1.5418 A).

**Table 5**

| 2-theta degrees | Intensity (%) |
|---|---|
| 7.9 | 6.9 |
| 8.4 | 39.1 |
| 9.0 | 100 |
| 11.0 | 18.5 |
| 12.0 | 6.1 |
| 13.2 | 27.0 |
| 14.7 | 7.2 |
| 15.8 | 10.6 |
| 17.1 | 3.3 |
| 18.0 | 18.9 |
| 19.2 | 15.0 |
| 19.9 | 6.5 |
| 20.6 | 8.1 |
| 21.2 | 5.7 |
| 22.3 | 8.2 |
| 22.8 | 18.1 |
| 24.0 | 80.3 |
| 25.4 | 11.6 |
| 27.1 | 18.1 |
| 28.1 | 21.3 |
| 28.5 | 11.0 |
| 29.0 | 10.6 |
| 29.6 | 5.6 |
| 30.6 | 2.4 |

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### EXAMPLES

Thermal Gravimetric Analyses (TGA) were performed on a Mettler-Toledo TGA-851e thermobalance. Experimental conditions: alumina crucibles of 70 µL volume, atmosphere of dry nitrogen with 50 mL/min flow rate, heating rate of 10°C/min.

PXRD analyses: The powder samples were sandwiched between polyester films of 10 micrometers of thickness or polyamide (kapton) films of 15 micrometers of thickness and analysed in a PANalytical X'Pert PRO MPD q/q powder diffractometer of 240 millimetres of radius, in a configuration of convergent beam with a focalizing mirror and a flat sample transmission geometry, in the following experimental conditions: Cu Kα radiation (λ = 1.5418 A); Work power: 45 kV and 40 mA; Incident beam slits defining a beam height of 0.4 millimetres; Incident and diffracted beam 0.02 radians Soller slits; PIXcel detector: Active length = 3.347 °; 2θ/θ scans from 2 to 40 °2θ with a step size of 0.026 °2θ and a measuring time of 75 seconds per step.

Karl Fischer analyses: water determination was carried out on a 831 Karl Fischer Coulometer with an 774 Oven Sample Processor. Solid sample is heated in the oven, the released water is transported in a dry stream of carrier gas to the Karl Fischer titration cell with a generator electrode without diaphragm, where it is determined with Hydranal Coulomat E reactive.

### Example 1: Strontium ranelate Form X

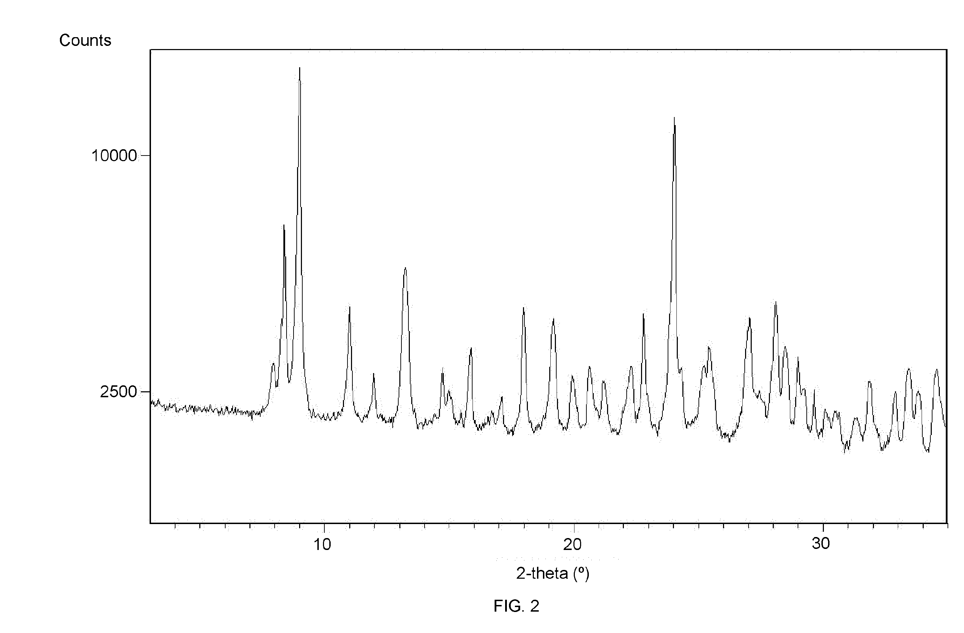

Strontium ranelate obtained according to the process described in example 1 second method (d) of EP 415850 was washed with water and dried in a forced-air oven at 20-25°C to a constant weight (503 mg). The resulting product was suspended in acetone (200 mL) at room temperature during 3 days. The green-yellow solid obtained was filtered and dried at 20-25°C under vacuum (7 mbar) for 6 h. The dried product was analysed by PXRD and TGA. Analysis of the PXRD confirmed that the solid form obtained was strontium ranelate Form X (FIG. 2). TGA shows a weight loss of 13.4% from 30 °C to 250 °C.

### Example 2: Strontium ranelate Form X

Strontium ranelate obtained according to the process described in example 1 second method (d) of EP 415850 was washed with water and dried in a forced-air oven at 20-25°C to a constant weight (10.2 mg). The resulting product was suspended in ethanol (10 mL) at room temperature during one week. The green-yellow solid obtained was filtered and dried at 20-25°C under vacuum (7 mbar) for 6 h. The dried product was analysed by PXRD. It was confirmed that the solid form obtained was strontium ranelate Form X.

### Example 3-9: Strontium ranelate Form X

Examples 3 to 9 were carried out according to the process described in example 2 but using different solvents (in each case, the amount of solvent is 10 mL) as shown in the following table.

| Example | Strontium ranelate (mg) | Solvent |
|---|---|---|
| 3 | 10.3 | 2-propanol |
| 4 | 10.4 | acetonitrile |
| 5 | 10.0 | Acetone |
| 6 | 10.1 | Dimethylformamide |
| 7 | 10.3 | Dimethylsulfoxide |
| 8 | 11.1 | Tetrahydrofuran |
| 9 | 10.2 | Dioxane |

### Example 10: Strontium ranelate Form X

Strontium ranelate obtained according to the process described in example 1 second method (d) of EP 415850 was washed with water and dried in a forced-air oven at 20-25°C to a constant weight (500 mg). The resulting product was washed with cold acetone (50 mL), filtered and dried at 20-25°C under vacuum (7 mbar) for 6 h. The dried product was analysed by PXRD and TGA. It was confirmed that the solid form obtained was strontium ranelate Form X. Analysis of TGA shows a weight loss of 13.4% from 30 °C to 248 °C.

### Example 11: Strontium ranelate Form X

Strontium ranelate obtained according to the process described in the second method (d) of patent EP 415850 (10.9 g) was washed with water (27.5 mL) at 20-25°C, after that was washed with acetone (9 x 8.5 mL). The resulting product was dried under vacuum at 20-25°C until obtaining a product with KF=13.3%.

### Comparative Example 12: Strontium ranelate Hydrate-1

Strontium ranelate obtained according to the process described in example 1 second method (d) of EP 415850 (23.3 g) was washed with water (2 x 60 mL) at 20-25°C, after that was washed several times with acetone (3 x 60 mL). The resulting product was dried in a forced-air oven at 20-25°C to a constant weight (20.5 g). The solid obtained was analysed by PXRD. It was confirmed that the solid form obtained was strontium ranelate Hydrate-1.

### Example 13: Strontium ranelate in form of a mixture of crystalline Form X and crystalline Hydrate-1

Strontium ranelate obtained according to the process described in the second method (d) of patent EP 415850 (10.9 g) was washed with water (27.5 mL) at 20-25°C, after that was washed with acetone (9 x 8.5 mL). The resulting product was dried under vacuum at 20-25°C until obtaining a product with KF=15.1 %.

### Example 14: Strontium ranelate in form of a mixture of crystalline Form X and crystalline Hydrate-1

Strontium ranelate obtained according to the process described in the second method (d) of patent EP0415850 (10.9 g) was washed with water (27.5 mL) at 20-25°C, after that was washed with acetone (9 x 8.5 mL). The resulting product was dried under vacuum at 20-25°C until obtaining a product with KF=16.2%.

### Example 15: Polymorphic stability

The polymorphic stability of strontium ranelate in the form of a mixture of crystalline Form X and crystalline Hydrate-1 was compared to the stability of strontium ranelate Hydrate-1.

For this purpose, the mixture of Example 13 and Hydrate-1 as obtained in Comparative Example 12 were placed separately in closed vials and left stand under room temperature for a period of time. It was found that the mixture of Example 13 was stable under the tested conditions after 4 months and that no transformation was observed. On the contrary, Hydrate-1 as obtained in Comparative Example 12 was not stable under the tested conditions and after 2 months was transformed to a mixture of Hydrate-1 and Hydrate-2. Hydrate-2 corresponds to the crystalline form identified in the experimental data filed by the opponent on 14.07.2009 during the opposition of EP 1642897. After three months the same mixture (Hydrate-1 + Hydrate-2) was detected. As regards the stability of crystalline Hydrate-1, the same results were obtained under an inert atmosphere (nitrogen) or under anhydrous conditions (silica).

### Example 16:

Strontium ranelate (2.4g), as the mixture of Example 14 or as Hydrate-1 (Comparative Example 12), was added to a cup containing 100 mL of purified water. The mixture was stirred with a spoon. The resulting suspension was poured into a 100 mL measuring cylinder. Flocculation was observed over the time and the following results were obtained:

| Time | Height (in mL) of the opalescent part | |
|---|---|---|
| | Comparative Example 12 | Example 14 |
| 1 min | 100 | 96 |
| 5 min | 55 | 81 |
| 15 min | 10 | 45 |
| 24 h | 7 | 10 |

### REFERENCES CITED IN THE APPLICATION

EP 415850
WO 2006/035122
WO 2010/034806
IN 2010CH01267
IN 2010CH03829
WO 2011/107454
CN 101108845
CN 101108845

## Claims

1. Strontium ranelate in a hydrated form, having a water content of from 14 to 19% ± 0.3% by weight.

2. Strontium ranelate according to claim 1, which is in the form of a mixture of crystalline Form X and crystalline Hydrate-1, wherein
crystalline Form X shows a X-ray diffractogram that comprises characteristic peaks at 8.8, 9.0, 10.8, 13.1, 14.3, 14.7, 15.0, 17.6, 19.8, 25.0 ± 0.2 degrees 2 theta measured in a X-ray diffractometer with Cu Kα radiation (1.5418 A), and
crystalline Hydrate-1 shows a X-ray diffractogram that comprises characteristic peaks at 7.9, 8.4, 9.0, 11.0, 13.2, 15.8, 18.0, 19.2, 22.8, 24.0 ± 0.2 degrees 2 theta measured in a X-ray diffractometer with Cu Kα radiation (1.5418 A).

3. Strontium ranelate according to any of the claims 1-2, wherein the water content is of from 15 to 17% ± 0.3% by weight.

4. Strontium ranelate according to any of the claims 1-3, wherein crystalline Form X is present in an amount of from 60 to 80% by weight, and crystalline Hydrate-1 is present in an amount of from 20 to 40% by weight, being the sum of both crystalline forms 100% by weight.

5. Strontium ranelate according to claim 4, which shows a X-ray diffractogram that comprises characteristic peaks at 7.9, 8.3, 8.8, 9.0, 9.1, 10.8, 13.2, 17.6, 18.0, 24.0 ± 0.2 degrees 2 theta measured in a X-ray diffractometer with Cu Kα radiation (1.5418 A).

6. Strontium ranelate according to any of the claims 1-3, wherein crystalline Form X is present in an amount of from 20 to 40% by weight, and crystalline Hydrate-1 is present in an amount of from 60 to 80 by weight, being the sum of both crystalline forms 100% by weight.

7. Strontium ranelate according to claim 6, which shows a X-ray diffractogram that comprises characteristic peaks at 7.8, 8.3, 8.8, 8.9, 11.0, 13.2, 17.6, 18.0, 19.2, 24.0 ± 0.2 degrees 2 theta ± 0.2 degrees 2 theta measured in a X-ray diffractometer with Cu Kα radiation (1.5418 A).

8. Process for the preparation of the crystalline strontium ranelate as defined in any of the claims 1-7, comprising:
a) providing strontium ranelate, having a X-ray diffractogram that comprises characteristic peaks at 7.6, 8.4, 8.6, 9.0, 13.1, 18.0, 19.1, 22.8, 23.1, 24.1, 25.6, 26.9, 28.2, 30.1 degrees 2 theta measured in a X-ray diffractometer with Cu Kα radiation (1.5406 A); and
b) drying the product of the previous step under vacuum at 20-25 °C until a water content of from 14 to 19% ± 0.3% by weight.

9. The process according to claim 8, further comprising the steps of:
i) washing the product of step a) with water;
ii) optionally washing the product of step i) with an organic solvent; and.
iii) isolating the product obtained in the previous step from the medium.

10. A pharmaceutical composition comprising a therapeutically effective amount of strontium ranelate as defined in any of the claims 1-7, together with one or more pharmaceutically acceptable excipients or carriers.

11. Strontium ranelate as defined in any of the claims 1-7, for use in the treatment of a bone disease, preferably osteoporosis or arthrosis.

12. Strontium ranelate in a hydrated form, having a water content of from 12.7 to 13.3% ± 0.3% by weight.

13. Strontium ranelate according to claim 12, which is in crystalline Form X and shows a X-ray diffractogram that comprises characteristic peaks at 8.8, 9.0, 10.8, 13.1, 14.3, 14.5, 14.7, 15.0, 15.6, 17.6, 19.8, 21.5, 21.8, 22.6, 23.6, 24.3, 25.0, 26.5, 26.8, 27.1, 28.2, 29.3, 30.0, 31.6 ± 0.2 degrees 2 theta measured in a X-ray diffractometer with Cu Kα radiation (1.5418 A).

14. Process for the preparation of the crystalline strontium ranelate as defined in claim 13, comprising:
a) providing strontium ranelate, having a X-ray diffractogram that comprises characteristic peaks at 7.6, 8.4, 8.6, 9.0, 13.1, 18.0, 19.1, 22.8, 23.1, 24.1, 25.6, 26.9, 28.2, 30.1 degrees 2 theta measured in a X-ray diffractometer with Cu Kα radiation (1.5406 A); and
b) drying the product of the previous step under vacuum at 20-25 °C until a water content of 13.3% ± 0.3% by weight.

15. The process according to claim 14, further comprising the steps of:
i) providing a suspension of the product of step a) in an organic solvent; and
ii) isolating the product of step i) from the medium; or alternatively further comprising the steps of:
i') washing the product of step a) with water;
ii') optionally washing the product of step i') with an organic solvent; and.
iii') isolating the product obtained in the previous step from the medium.
